# EUROPÄISCHE PATENTSCHRIFT

(11) **EP 3 443 920 B1**
(45) Veröffentlichungstag und Bekanntmachung des Hinweises auf die Patenterteilung: **04.03.2020**
(21) Anmeldenummer: 18187937.0
(22) Anmeldetag: 08.08.2018
(51) Int. Cl.: A61B 18/14, A61B 17/00, A61B 18/16

(54) **BIPOLARES RESEKTOSKOP**
BIPOLAR RESECTOSCOPE
RÉSECTOSCOPE BIPOLAIRE

(30) Priorität: 18.08.2017 DE 102017118885
(43) Veröffentlichungstag der Anmeldung: 20.02.2019
(73) Patentinhaber: BOWA-electronic GmbH & Co. KG, 72810 Gomaringen (DE)
(72) Erfinder: Hluchy, Heinz, 72116 Mössingen (DE)
(74) Vertreter: Schneider, Peter Christian

(56) Entgegenhaltungen:
- EP-A1- 2 767 250
- EP-B1- 1 163 886
- DE-A1- 2 521 719
- DE-A1-102013 001 156

## Beschreibung

### Gebiet der Erfindung

Die Erfindung betrifft ein bipolares Resektoskop, umfassend
- einen Schaft, der an seinem distalen Ende einen Isoliereinsatz aus einem elektrisch nicht leitenden Material aufweist, wobei der Isoliereinsatz an seinem freiliegenden, dem Schaft abgewandten distalen Ende, einen in vertikaler Richtung unterhalb seiner Längsachse angeordneten Einlaufbereich für eine in dem Schaft längsverschieblich anordenbare Schneidelektrode aufweist, und wobei der Isoliereinsatz einen freiliegenden, elektrisch leitenden Bereich einer Neutralelektrode aufweist, die mit einem passiven Elektrodenanschluss am proximalen Ende des Schaftes in elektrischer Verbindung steht,
- ein in dem Schaft anordenbares Arbeitselement in dem die aktive Schneidelektrode längsverschieblich anordenbar ist, die an ihrem dem distalen Ende abgewandten proximalen Ende mit einem aktiven Elektrodenanschluss am proximalen Ende des Arbeitselementes in elektrischer Verbindung steht, und der ein Optikführungsrohr zur Aufnahme einer in den Arbeitselement einsetzbaren Optik aufweist, und
- die aktive Schneidelektrode.

### Stand der Technik

Aus der WO 2014/114600 A1 / DE 10 2013 001 156 A1 ist ein bipolares Resektoskop bekannt, das einen Schaft umfasst, der an seinem distalen Ende einen Isoliereinsatz aus einem elektrisch nicht leitenden Material aufweist. Dabei weist der Isoliereinsatz an seinem freiliegenden, dem Schaft abgewandten distalen Ende einen unterhalb seiner Längsachse angeordneten Einlaufbereich für eine in dem Schaft längsverschieblich anordenbare Schneidelektrode auf. Der Isoliereinsatz weist einen freiliegenden, elektrisch leitenden Bereich einer Neutralelektrode auf, die mit einem passiven Elektrodenanschluss am proximalen Ende des Schaftes in elektrischer Verbindung steht. Weiter umfasst das bipolare Resektoskop ein in dem Schaft anordenbares Arbeitselement, in dem die aktive Schneidelektrode längsverschieblich anordenbar ist, die an ihrem dem distalen Ende angewandten proximalen Ende mit einem aktiven Elektrodenanschluss am proximalen Ende des Arbeitselementes in elektrischer Verbindung steht. Schließlich umfasst das bekannte bipolare Resektoskop eine in ein Optikführungsrohr des Arbeitselementes einsetzbare Optik.

Nach einer ersten Ausführungsform des bekannten bipolaren Resektoskopes weist dessen Isoliereinsatz als freiliegenden, elektrisch leitenden Bereich einer Neutralelektrode eine freiliegenden Elektrodenfläche auf, die an der der Längsachse des Isoliereinsatzes radial zugewandten Innenseite angeordnet ist und radial nach außen hin elektrisch isoliert ist.

Nachteilig dabei ist, dass die Elektrodenfläche umlaufend ausgebildet ist und im Einlaufbereich des Isoliereinsatzes ein unerwünscht hoher Anstieg der Stromdichte an der Schneidschlinge der aktiven Schneidelektrode auftreten kann, wogegen im ausgefahrenem Zustand der Schneidschlinge beim Eintauchen in das zu schneidende Gewebe eine eher zu niedrige Stromdichte mit schwierigem Zündpunkt zur Plasmaentstehung.

Nach einer weiteren Ausführungsform des bekannten bipolaren Resektoskopes ist die freiliegende Elektrodenfläche des Isoliereinsatzes an der der Längsachse des Isoliereinsatzes radial abgewandten Außenseite angeordnet und nach innen hin elektrisch isoliert. Dies hat den Nachteil, dass einerseits im Einlaufbereich der Abstand der Schneidschlinge der aktiven Schneidelektrode zur Elektrodenfläche der Neutralelektrode sehr veränderlich ist und höhere Leistungen benötigt werden und dass zum anderen die Stromdichte auch von der Position des Schaftes in der Harnröhre des Patienten besonders abhängt.

Weiterhin ist aus der DE 25 21 719 A1 ein bipolares Resektoskop bekannt, das ebenfalls als aktive Elektrode eine schlingenförmige Schneidelektrode aufweist, die über ein Arbeitselement längsverschieblich in einem Endoskopschaft gelagert ist. Als passive Elektrode oder Neutralelektrode dient ein zwischen einem distalen Isoliereinsatz und dem distalen Ende eines Endoskopschaftes angeordnetes, elektrisch leitendes Rohrstück.

Nachteilig dabei ist, dass das elektrisch leitende Rohrstück vom distalen Ende des Isoliereinsatzes zur Vermeidung eines direkten Kontaktes der Schneidschlinge der Schneidelektrode außerhalb des Einlaufbereiches angeordnet ist. Dies führt in Verbindung mit dem umliegenden Gewebe abhängig von der Positionierung des Schaftes in der männlichen Harnröhre ebenfalls zu unerwünscht unterschiedlichen Stromdichten.

Aus der EP 1 163 886 B1 ist ein bipolares Resektoskop bekannt, das einen Schaft umfasst, der an seinem distalen Ende einen Isoliereinsatz aus einem elektrisch nicht leitenden Material aufweist. Dabei weist der Isoliereinsatz an seinem freiliegenden, dem Schaft abgewandten distalen Ende einen unterhalb seiner Längsachse angeordneten Einlaufbereich für eine in dem Schaft längsverschieblich anordenbare Schneidelektrode auf. Weiter umfasst das bipolare Resektoskop ein in dem Schaft anordenbares Arbeitselement, in dem die aktive Schneidelektrode längsverschieblich anordenbar ist, die an ihrem dem distalen Ende angewandten proximalen Ende mit einem aktiven Elektrodenanschluss am proximalen Ende des Arbeitselementes in elektrischer Verbindung steht. Schließlich umfasst das bekannte bipolare Resektoskop eine in ein Optikführungsrohr des Arbeitselementes einsetzbare Optik. Der elektrisch leitende Bereich einer Neutralelektrode des bekannten bipolaren Resektoskopes ist am distalen Ende der aktiven Schneidelektrode angeordnet und überspannt zwei parallel zueinander verlaufende gabelförmige Führungsrohre der aktiven Schneidelektrode dachförmig.

Nachteilig dabei ist, dass die Fläche der Neutralelektrode relativ großflächig sein muss und mit der Schneidschlinge ausgefahren wird, so dass sie das Blickfeld der Optik unerwünscht stört. Auch muss die Leitung der Neutralelektrode zusammen mit der Leitung der Aktivelektrode in der Schneidelektrode zu derem proximalen Ende geführt werden. Die ausgefahrene Elektrodenfläche der Neutralelektrode kann je nach Stellung des Resektoskopes in der Harnröhre umliegendes Gewebe kontaktieren und den Stromfluss unerwünscht beeinflussen. Aus der EP 2 767 250 sind Kontaktröhrchen elektrochirurgischer Resektoskope bekannt.

### Aufgabenstellung

Es ist die Aufgabe der vorliegenden Erfindung, ein gattungsgemäßes bipolares Resektoskop derart weiterzubilden, dass einerseits die passive Neutralelektrode mit ausreichende großer Elektrodenfläche möglichst nah an der aktiven Schneidelektrode angeordnet ist, dass andererseits weitgehend unabhängig von der Positionierung des Schaftes in der männlichen Harnröhre unter Beachtung der elektrischen Sicherheit ausreichend hohe Stromdichten an der Schneidschlinge in Verbindung mit dem zu resezierenden Gewebe entstehen.

### Darlegung der Erfindung

Die Aufgabe bezüglich des bipolaren Resektoskopes wird in Verbindung mit den Merkmalen des Oberbegriffs von Anspruch 1 dadurch gelöst, dass der quer zur Längsachse nach außen hin freiliegende Bereich der passiven Neutralelektrode einen nach innen hin durchgängig elektrisch leitenden Dachbereich des Isoliereinsatzes bildet, dass der freiliegende Bereich der passiven Neutralelektrode im Einlaufbereich auf den Dachbereich des Isoliereinsatzes beschränkt ist, dass der Dachbereich quer zur Längsachse des Isoliereinsatzes in vertikaler Richtung oberhalb des Einlaufbereiches angeordnet ist, und dass der Dachbereich sich in Richtung der Längsachse über den Einlaufbereich des Isoliereinsatzes erstreckt, dass der Isoliereinsatz im Einlaufbereich an seiner Innenwandung zwei einander gegenüberliegende und parallel zur Längsachse verlaufende, freiliegende elektrisch leitende Kontaktflächen der Neutralelektrode aufweist, die bei in dem Schaft angeordneter Schneidelektrode mit jeweils einem benachbarten Kontaktröhrchen einer zwei parallele, elektrisch leitende Kontaktröhrchen aufweisenden Gabel, die eine Schneidschlinge der Schneidelektrode aufspannt, in metallischem, elektrisch leitenden Kontakt zur Neutralelektrode stehen, und dass die elektrisch leitenden Kontaktröhrchen gegenüber der Gabel elektrisch isoliert sind.

Dadurch, dass der freiliegende Bereich der passiven Neutralelektrode einen durchgängig elektrisch leitenden Dachbereich des Isoliereinsatzes bildet, werden insbesondere die im Einlaufbereich beim Resezieren von Gewebe entstehenden Stromdichten unabhängiger von der Positionierung des Schaftes in der männlichen Harnröhre. Dadurch, dass sich der freiliegende Bereich der passiven Neutralelektrode auf den Dachbereich des Isoliereinsatzes beschränkt, wird insbesondere die elektrische Sicherheit im Einlaufbereich des Isoliereinsatzes gegenüber der Schneidschlinge der Schneidelektrode erhöht. Gleichzeitig wird die Abhängigkeit der Stromdichte von der Positionierung des Schaftes beim Resezieren weiter verringert. Die beiden parallel zueinander angeordneten elektrisch leitenden Kontaktröhrchen sind Teile einer Gabel, die eine Schneidschlinge am distalen Ende der Schneidelektrode aufspannt, wobei die Kontaktröhrchen gegenüber der Gabel elektrisch isoliert sind.

Durch die elektrisch leitenden Kontaktröhrchen und ihrem elektrischen Kontakt zur Neutralelektrode wirken die Kontaktröhrchen als Vergrößerung des freiliegenden Bereiches der Neutralelektrode, so dass Teile der freiliegenden Elektrodenfläche der Neutralelektrode nach innen, näher zur aktiven Schneidelektrode hin verlegt werden. Unabhängig von der jeweiligen Positionierung der längsverschieblichen Schneidelektrode weisen die von den Kontaktröhrchen gebildeten Teilflächen der Neutralelektrode jeweils den gleichen Abstand auf, da sie sich als fixer Bestandteil der Schneidelektrode mit dieser mitbewegen. Neben verbesserter und konstanterer Stromdichten führt diese Anordnung zugleich zu einem verbesserten Zündpunkt zur Plasmaerzeugung beim Beginn des jeweiligen Reseziervorganges.

Gemäß einer bevorzugten Ausführungsform der Erfindung werden die freiliegenden, elektrisch leitenden Kontaktflächen der Neutralelektrode des Isoliereinsatzes außerhalb des Dachbereiches und zum proximalen Ende des Isoliereinsatzes hin von einer nach innen hin freiliegenden und umlaufenden Kontaktfläche der Neutralelektrode gebildet. Dadurch wird ohne Benachteiligung der elektrischen Sicherheit die Fläche der passiven Neutralelektrode im proximalen Bereich des Isoliereinsatzes erhöht.

Nach einer weiteren bevorzugten Ausführungsform der Erfindung weisen die elektrisch leitenden Kontaktröhrchen zu einer in das Arbeitselement eingesetzten Optik hin Längsöffnungen auf, die die darunterliegende Isolierschicht freigeben. Diese Längsöffnungen der Kontaktröhrchen verhindern, dass bei evtl. durch Belastung verbogener Elektrode ein metallischer, d.h. elektrisch leitender Kontakt zu der eingesetzten Optik entstehen kann.

Nach einer weiteren bevorzugten Ausführungsform der Erfindung weist die Schneidelektrode im Bereich der Gabel ein am distalen Ende der in das Arbeitselement eingesetzten Optik geführtes Führungsrohr auf, das gegenüber den Kontaktröhrchen und gegenüber der Gabel elektrisch isoliert ist. Das Führungsrohr der Schneidelektrode dient in an sich bekannter Weise zur Stabilisierung des distalen Endes der Schneidelektrode gegenüber der Optik und damit gegenüber dem Schaft. Dadurch, dass das Führungsrohr der Schneidelektrode gegenüber den Kontaktröhrchen und gegenüber der Gabel elektrisch isoliert oder aus einem elektrisch nicht leitenden Material ausgebildet ist, wird ein elektrischer Schluss zur Optik und weiter zum restlichen Instrument verhindert.

Gemäß einer weiteren bevorzugten Ausführungsform der Erfindung weist die Neutralelektrode in ihrem Umfang außerhalb des Dachbereiches und der Kontaktflächen oder Kontaktfläche sowohl an ihrer Außenfläche als auch an ihrer Innenfläche eine elektrisch nicht leitende Beschichtung auf. Zur Herstellung des Isoliereinsatzes kann es einfach und kostengünstiger sein, die Neutralelektrode umlaufend anzuordnen und sie dann außerhalb ihres Dachbereiches sowohl an ihrer Außenfläche als auch an ihrer Innenfläche mit einer nichtleitenden Beschichtung zu versehen.

Auch kann die Neutralelektrode an ihrer am distalen Ende des Isoliereinsatzes angeordneten Stirnfläche außerhalb des Dachbereiches eine elektrisch nicht leitende Beschichtung aufweisen.

Nach einer weiteren bevorzugten Ausführungsform der Erfindung ist die Neutralelektrode des Isoliereinsatzes in Längsrichtung, das heißt in Richtung der Längsachse des Isoliereinsatzes, gegenüber dem distalen Ende des Schaftes nicht leitend ausgebildet.

Gemäß einer weiteren bevorzugten Ausführungsform der Erfindung ist mindestens der Einlaufbereich des Isoliereinsatzes durch eine Keramik oder eine Keramikschicht aus Zirconiumdioxid geschützt. Dies erhöht insbesondere die Standzeit des Isoliereinsatzes an der Schneidkante im Einlaufbereich gegenüber der Schneidschlinge.

Gemäß einer weiteren bevorzugten Ausführungsform der Erfindung ist der Isoliereinsatz aus einem Kunststoff ausgebildet und die Neutralelektrode aus einem metallischen Werkstoff ausgebildet.

Nach einer weiteren bevorzugten Ausführungsform der Erfindung ist der Isoliereinsatz aus einer Keramik ausgebildet, wobei die die Neutralelektrode aus einer metallisierten Keramik ausgebildet ist.

Nach einer weiteren bevorzugten Ausführungsform der Erfindung ist der Schaft aus einem elektrisch leitenden Metall ausgebildet. Dies ist insbesondere auch in Verbindung mit einem Außenschaft aus einem elektrisch leitenden Material von Vorteil.

Gemäß einer weiteren bevorzugten Ausführungsform der Erfindung ist der Schaft mindestens auf seiner Außenfläche mit einem elektrisch nicht leitenden Material beschichtet.

Nach einer weiteren bevorzugten Ausführungsform der Erfindung ist der Schaft in einem Außenschaft einsteckbar, wobei zwischen dem Außenschaft und dem eingesetzten Schaft ein Absaugkanal gebildet wird. Der Außenschaft und der Schaft, der in diesem Fall einen Innenschaft bildet, sind dabei in dem Fachmann bekannter Weise miteinander verriegelbar. Der Schaft und der Außenschaft bilden dabei einen sogenannten Dauerspülschaft mit kontinuierlicher Spülung, bei dem über den Schaft Spülflüssigkeit zugeführt und Flüssigkeit über den zwischen Schäften gebildeten Absaugkanal abgesaugt werden kann.

Bevorzugt ist das distale Ende des Außenschaftes gegenüber dem distalen Ende des Isoliereinsatzes des Schaftes in proximaler Richtung im Bereich des Isoliereinsatzes zurückgesetzt. Dabei kann der Außenschaft an seinem distalen Ende eine Mehrzahl von Rückflussöffnungen aufweisen.

Die Rückflussöffnungen können aber auch von Vertiefungen im Bereich des distalen Endes des Schaftes gebildet werden, die teilweise vom distalen Ende des Außenschaftes abgedeckt werden.

Weitere Merkmale und Vorteile der Erfindung ergeben sich aus der nachfolgenden speziellen Beschreibung und den Zeichnungen.

### Kurzbeschreibung der Zeichnungen

Es zeigen:
- Figur 1:: eine Seitenansicht eines bipolaren Resektoskopes mit gestrichelt angedeuteter Optik;
- Figur 2:: eine Seitenansicht des distalen Endes von Fig. 1 im Schnitt und vergrößerter Darstellung;
- Figur 3:: eine Seitenansicht des distalen Endes eines bipolaren Resektoskopes einer zweiten Ausführungsform im Schnitt und vergrößerter Darstellung;
- Figur 4:: eine Seitenansicht des distalen Endes von Fig. 3 ohne Optik;
- Figur 5:: eine Seitenansicht des distalen Endes von Fig. 4 mit gestrichelt dargestellter Schneidelektrode im ausgefahrenen Zustand;

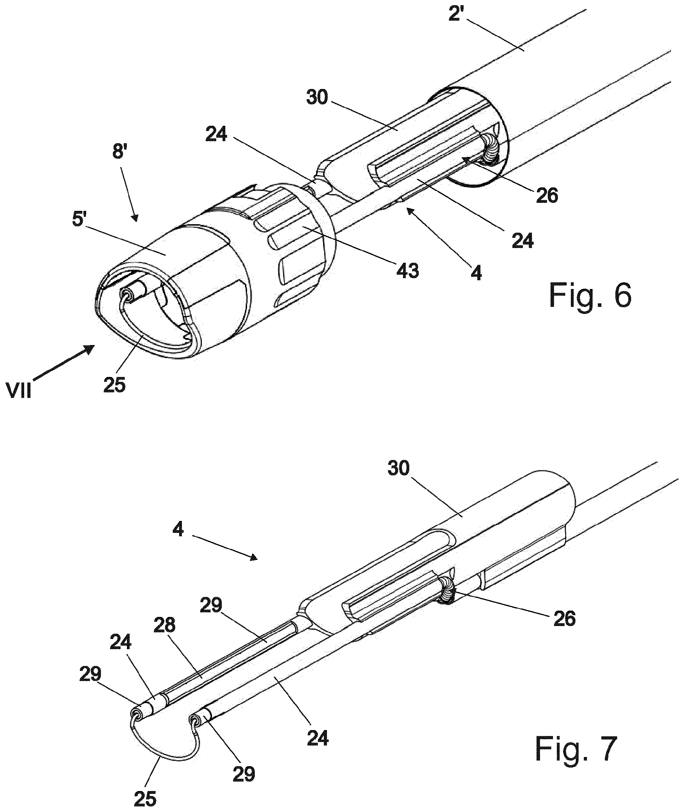

- Figur 6:: eine räumliche Darstellung des distalen Endes von Fig. 4 teilweise aufgeschnitten;
- Figur 7:: eine räumliche Darstellung des distalen Endes der Schneidelektrode von Fig. 5;

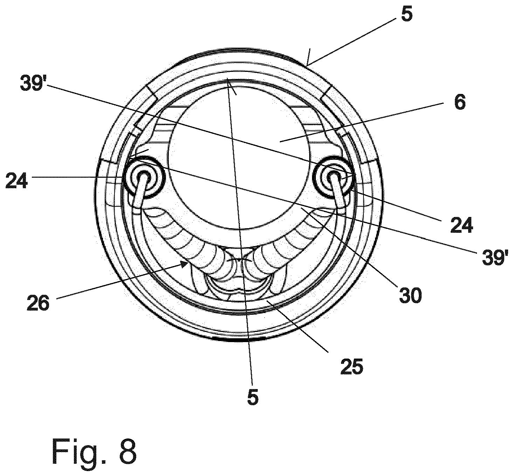

- Figur 8:: eine Vorderansicht des distalen Endes von Fig. 6 aus Richtung VIII.

### Beschreibung bevorzugter Ausführungsformen

Ein bipolares Resektoskop 1 besteht im Wesentlichen aus einem Schaft 2, einem Arbeitselement 3, einer aktiven Schneidelektrode 4, einer passiven Neutralektrode 5 und einer Optik 6.

Der Schaft 2 weist an seinem dem Patienten zugewandten distalen Ende 7 einen Isoliereinsatz 8 auf. In den Schaft 2 ist von dessen dem distalen Ende 7 abgewandten proximalen Ende 9 her das Arbeitselement 3 mit der Schneidelektrode 4 einsetzbar und mit dem Schaft 2 verrastbar.

Das Arbeitselement 3 weist ein Führungsrohr 10 auf, an dessen dem Operateur zugewandten proximalen Ende 11 ein Anschlussstück 12 zum Anschluss der Optik 6, die in dem Optikführungsrohr 10 geführt wird, angeordnet ist. Das Arbeitselement 3 weist einen Fingergriff 13 und einen in distaler Richtung vorlagerten Anschlusskonus 14 auf, in dem das Arbeitselement 3 mit einem das proximale Ende des Schaftes 2 bildenden Hauptkörper 15 verriegelbar ist.

Auf dem Optikführungsrohr 10 ist ein längsverschieblicher Schiebekörper 16 gelagert. Über ein federndes Gelenk 17 ist der Schiebekörper 16 mit dem Anschlussstück 12 verbunden und kann über einen Daumenring 18 gegen die Federkraft des Gelenkes 17 in Richtung Fingergriff 13 gedrückt werden. Der Schiebekörper 16 weist eine Aufnahmeöffnung zur Aufnahme eines proximalen Endes 19 der aktiven Schneidelektrode 4 auf. Der Schiebekörper 16 weist einen Führungskanal 20 auf, mit der Schiebekörper 16 auf dem Optikführungsrohr 10 geführt wird, wobei die Längsachse 21 des Optikführungsrohr 10 mit der Längsachse des Führungskanals 20 zusammenfällt. Quer zur Längsachse 21 weist der Schiebekörper 16 eine Steckeraufnahme 22 für einen nicht dargestellten Stecker auf, der an einem instrumentenseitigen Ende eines Hochfrequenzkabels mit einem aktiven Anschluss eines ebenfalls nicht dargestellten Hochfrequenzgenerators verbindbar ist. Entsprechend ist die Neutralelektrode 5 an ihrem proximalen Ende mit einem zweiten Anschluss des Hochfrequenzgenerators verbindbar.

Die Schneidelektrode 4 ist in an sich bekannter Weise an ihrem distalen Ende 23 gabelförmig geteilt und weist etwa parallel zur Längsachse 21 zwei parallele Schlingenführungsrohre einer Gabel 26 auf, zwischen denen eine halbkreisförmige Schneidschlinge 25 aufgespannt ist. Erfindungsgemäß sind die Schlingenführungsrohre als elektrisch leitende Kontaktröhrchen 24 ausgebildet, die gegenüber der Gabel 26 elektrisch isoliert sind. Die Kontaktröhrchen 24 weisen zum distalen Ende 27 der in das Arbeitselement 3 eingesetzten Optik 6 hin jeweils eine Längsöffnung 28 auf, die ein Fenster bildet, das die darunter liegende Isolierschicht 29 der Gabel 26 freilegt.

Die Schneidelektrode 4 weist im Bereich der Gabel 26 ein am distalen Ende 27 der in das Arbeitselement 3 eingesetzten Optik 6 geführtes Führungsrohr 30 auf, das gegenüber den Kontaktröhrchen 24 und gegenüber der Gabel 26 elektrisch isoliert ist. Insbesondere kann das Führungsrohr 30 der Schneidelektrode 4 aus einem elektrisch nicht leitenden Material ausgebildet sein.

Der Isoliereinsatz 8 ist mit seinem proximalen Ende 31 in dem distalen Ende 7 des Schaftes 2, beispielsweise über eine Steckverbindung, angeordnet. Dabei fällt die Längsachse 33 des Isoliereinsatzes 8 mit der Längsachse 34 des Schaftes 2 zusammen. An seinem der Schneidschlinge 25 in vertikaler Richtung nach oben abgewandten Dachbereich 32 weist der Isoliereinsatz 8 zu seinem dem proximalen Ende 31 abgewandten distalen Ende 35 hin quer zur Längsachse 33 einen nach außen hin freiliegende Bereich der passiven Neutralelektrode 5 auf, der den nach innen hin durchgängig elektrisch leitenden Dachbereich 32 des Isoliereinsatzes 8 bildet. Dem Dachbereich 32 in vertikaler Richtung nach unten gegenüberliegend ist ein von dem Dachbereich 32 überdachter Einlaufbereich 36 angeordnet. Im Einlaufbereich 36 befindet sich eine Schneidkante 37 über die die Schneidschlinge 25 beim Zurückziehen der Schneidelektrode 4 in den Schaft 2 gleitet.

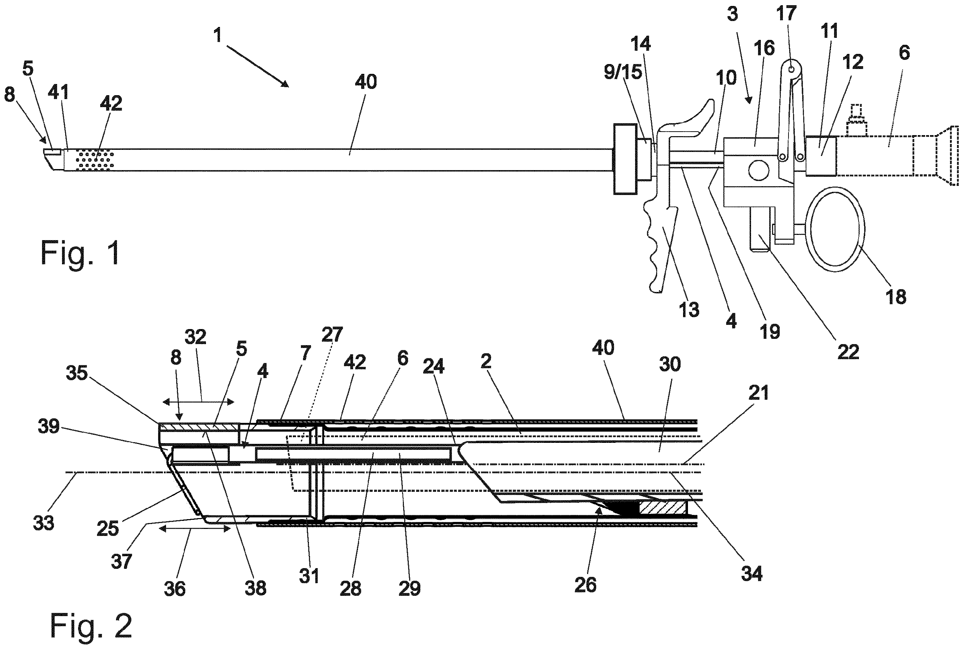

Entsprechend den Ausführungsbeispielen der Figuren 1 und 2 weist der Isoliereinsatz 8 im Einlaufbereich 36 an seiner Innenwandung 38 zwei einander gegenüberliegende und parallel zur Längsachse 33 verlaufende, freiliegende elektrisch leitende Kontaktflächen 39 der Neutralelektrode 5 auf, die bei in dem Schaft 2 angeordneter Schneidelektrode 4 mit jeweils dem benachbarten Kontaktröhrchen 24 der Schneidelektrode 4 in metallischem Kontakt stehen.

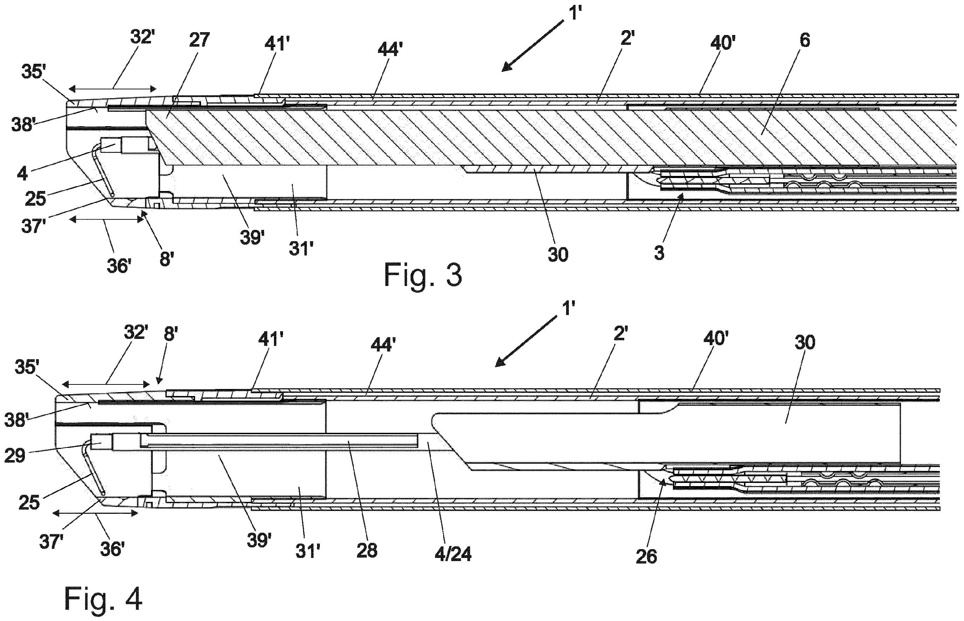

Entsprechend den Ausführungsbeispielen der Figuren 3 bis 5 weist der Isoliereinsatz 8' außerhalb des Dachbereiches 32' zum proximalen Ende 31' des Isoliereinsatzes 8' hin eine nach innen hin freiliegende und umlaufende Kontaktfläche 39' der Neutralelektrode 5' auf. Die Kontaktfläche 39' ist somit ring- oder hülsenförmig ausgebildet und geht in distaler Richtung in den Bereich der Neutralelektrode 5' im Dachbereich 32' über.

Entsprechend den Ausführungsbeispielen der Figuren 1 bis 4 ist der Schaft 2, 2' in einem Außenschaft 40, 40' angeordnet, wobei die beiden Schäfte 2, 2' und 40, 40' einen Dauerspülschaft mit kontinuierlicher Spülung bilden. Dabei ist das distale Ende 41, 41' des Außenschaftes 40, 40' gegenüber dem distalen Ende 35, 35' des Isoliereinsatzes 8, 8' in proximaler Richtung im Bereich des Isoliereinsatzes 8, 8' zurückgesetzt, also beabstandet. Zwischen dem Schaft 2, 2' und dem Außenschaft 40, 40' wird ein Absaugkanal 44, 44' gebildet.

Entsprechend Fig. 1 weist der Außenschaft 40 an seinem distalen Ende 41 eine Mehrzahl von Rückflussöffnungen 42 auf.

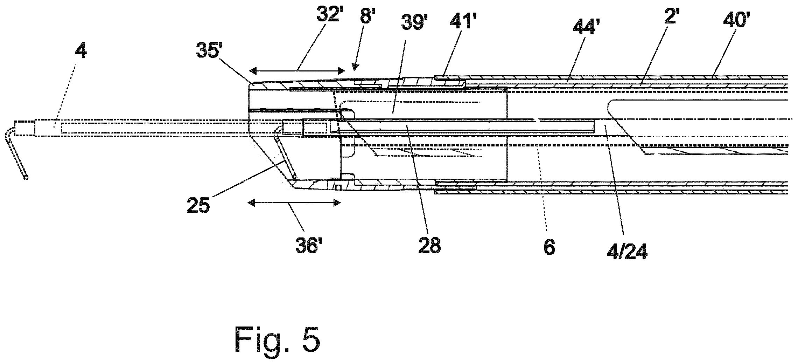

Entsprechend Fig. 5 weist der Schaft 2' an seinem distalen Ende 41' eine Mehrzahl von rillenartigen Vertiefungen 43' auf, die in Verbindung mit dem Außenschaft 40' Zuflussöffnungen zu dem Absaugkanal 44' bilden.

### Bezugszeichenliste

- 1, 1': bipolares Resektoskop
- 2, 2': Schaft
- 3: Arbeitselement
- 4: Schneidelektrode
- 5, 5': Neutralelektrode
- 6: Optik
- 7: distales Ende von 2
- 8, 8': Isoliereinsatz von 2
- 9: proximales Ende von 2
- 10: Optikführungsrohr von 3
- 11: proximales Ende von 3
- 12: Anschlussstück von 3
- 13: Fingergriff von 3
- 14: Anschlusskörper von 3
- 15: Hauptkörper von 2
- 16: Schiebekörper von 3
- 17: Gelenk von 3
- 18: Daumenring von 16
- 19: proximales Ende von 4
- 20: Führungskanal von 16
- 21: Längsachse von 10
- 22: Steckeraufnahme
- 23: distales Ende von 4
- 24: Kontaktröhrchen
- 25: Schneidschlinge
- 26: Gabel
- 27: distales Ende von 6
- 28: Längsöffnung von 24
- 29: Isolierschicht von 26
- 30: Führungsrohr von 4
- 31, 31': proximales Ende von 8
- 32, 32': Dachbereich von 8
- 33: Längsachse von 8
- 34: Längsachse von 2
- 35, 35': distales Ende von 8
- 36, 36': Einlaufbereich von 8
- 37, 37': Schneidkante von 8
- 38, 38': Innenwandung von 8
- 39, 39': Kontaktflächen
- 40, 40': Außenschaft
- 41, 41': distales Ende von 40
- 42: Rückflussöffnungen
- 43: Vertiefungen von 2'
- 44, 44': Absaugkanal

## Patentansprüche

1. Bipolares Resektoskop (1, 1'), umfassend
- einen Schaft (2, 2'), der an seinem distalen Ende (7) einen Isoliereinsatz (8, 8') aus einem elektrisch nicht leitenden Material aufweist, wobei der Isoliereinsatz (8, 8') an seinem freiliegenden, dem Schaft (2, 2') abgewandten distalen Ende (7), einen in vertikaler Richtung unterhalb seiner Längsachse (33) angeordneten Einlaufbereich (36, 36') für eine in dem Schaft (2, 2') längsverschieblich anordenbare Schneidelektrode (4) aufweist, und wobei der Isoliereinsatz (8, 8') einen freiliegenden, elektrisch leitenden Bereich einer Neutralelektrode (5, 5') aufweist, die mit einem passiven Elektrodenanschluss am proximalen Ende (7) des Schaftes (2, 2') in elektrischer Verbindung steht,
- ein in dem Schaft (2, 2') anordenbares Arbeitselement (3) in dem die aktive Schneidelektrode (4) längsverschieblich anordenbar ist, die an ihrem dem distalen Ende (23) abgewandten proximalen Ende (19) mit einem aktiven Elektrodenanschluss am proximalen Ende (11) des Arbeitselementes (3) in elektrischer Verbindung steht, und das ein Optikführungsrohr (10) zur Aufnahme einer in das Arbeitselement (3) einsetzbaren Optik (6) aufweist, und
- die aktive Schneidelektrode (4),
**dadurch gekennzeichnet,**
**dass** der quer zur Längsachse (33) des Isoliereinsatzes (8, 8') nach außen hin freiliegende Bereich der passiven Neutralelektrode (5, 5') einen nach innen hin durchgängig elektrisch leitenden Dachbereich (32, 32') des Isoliereinsatzes (8, 8') bildet,
**dass** der freiliegende Bereich der passiven Neutralelektrode (5, 5') im Einlaufbereich (36, 36') auf den Dachbereich (32, 32') des Isoliereinsatzes (8, 8') beschränkt ist, dass der Dachbereich quer zur Längsachse (33) des Isoliereinsatzes in vertikaler Richtung oberhalb des Einlaufbereiches (36, 36') angeordnet ist, und
**dass** der Dachbereich (32, 32') sich in Richtung der Längsachse (33) über den Einlaufbereich (36, 36') des Isoliereinsatzes (8, 8') erstreckt,
**dass** der Isoliereinsatz (8, 8') im Einlaufbereich (36, 36') an seiner Innenwandung (38, 38') zwei einander gegenüberliegende und parallel zur Längsachse (33) verlaufende, freiliegende elektrisch leitende Kontaktflächen (39, 39') der Neutralelektrode (5, 5') aufweist, die bei in dem Schaft (2, 2') angeordneter Schneidelektrode (4) mit jeweils einem benachbarten Kontaktröhrchen (24) einer zwei parallele, elektrisch leitende Kontaktröhrchen (24) aufweisenden Gabel (26), die eine Schneidschlinge (25) der Schneidelektrode (4) aufspannt, in metallischem, elektrisch leitenden Kontakt zur Neutralelektrode (5, 5') stehen, und
**dass** die elektrisch leitenden Kontaktröhrchen (24) gegenüber der Gabel (26) elektrisch isoliert sind.

2. Bipolares Resektoskop nach Anspruch 1,
**dadurch gekennzeichnet,**
**dass** die freiliegenden, elektrisch leitenden Kontaktflächen (39, 39') der Neutralelektrode (5, 5') des Isoliereinsatzes (8, 8') außerhalb des Dachbereiches (32, 32') zum proximalen Ende (31, 31') des Isoliereinsatzes (8, 8') hin von einer nach innen hin freiliegenden und umlaufenden Kontaktfläche (39, 39') der Neutralelektrode (5, 5') gebildet werden.

3. Bipolares Resektoskop nach Anspruch 1 oder 2,
**dadurch gekennzeichnet,**
**dass** die elektrisch leitenden Kontaktröhrchen (24) zu einer eingesetzten Optik (6) hin Längsöffnungen (28) aufweisen, die die darunterliegende Isolierschicht (29) freigeben.

4. Bipolares Resektoskop nach Anspruch 2 oder 3,
**dadurch gekennzeichnet,**
**dass** die Schneidelektrode (4) im Bereich der Gabel (26) ein am distalen Ende (23) der in das Arbeitselement (3) eingesetzten Optik (6) geführtes Führungsrohr (30) aufweist, das gegenüber den Kontaktröhrchen (24) und gegenüber der Gabel (26) elektrisch isoliert ist.

5. Bipolares Resektoskop nach Anspruch 4,
**dadurch gekennzeichnet,**
**dass** das Führungsrohr (30) der Schneidelektrode (4) aus einem elektrisch nicht leitenden Material ausgebildet ist.

6. Bipolares Resektoskop nach einem der Ansprüche 1 bis 5,
**dadurch gekennzeichnet,**
**dass** die Neutralelektrode (5, 5') in ihrem Umfang außerhalb des Dachbereiches (32, 32') und der Kontaktflächen (39, 39') oder Kontaktfläche (39, 39') sowohl an ihrer Außenfläche als auch an ihrer Innenfläche eine elektrisch nicht leitende Beschichtung aufweist.

7. Bipolares Resektoskop nach einem der Ansprüche 1 bis 6,
**dadurch gekennzeichnet,**
**dass** die Neutralelektrode (5, 5') an ihrer am distalen Ende (35, 35') des Isoliereinsatzes (8, 8') angeordneten Stirnfläche außerhalb des Dachbereiches (32, 32') eine elektrisch nicht leitende Beschichtung aufweist.

8. Bipolares Resektoskop nach einem der Ansprüche 1 bis 7,
**dadurch gekennzeichnet,**
**dass** die Neutralelektrode (5, 5') des Isoliereinsatzes (8, 8') in Längsrichtung gegenüber dem distalen Ende (35, 35') des Schaftes (2, 2') nicht leitend ausgebildet ist.

9. Bipolares Resektoskop nach einem der Ansprüche 1 bis 8,
**dadurch gekennzeichnet,**
**dass** mindestens der Einlaufbereich (36, 36') des Isoliereinsatzes (8, 8') durch eine Keramik aus Zirconiumdioxid geschützt ist.

10. Bipolares Resektoskop nach einem der Ansprüche 1 bis 9,
**dadurch gekennzeichnet,**
**dass** der Isoliereinsatz (8, 8') aus einem Kunststoff ausgebildet ist und
**dass** die Neutralelektrode (5, 5') aus einem metallischen Werkstoff ausgebildet ist.

11. Bipolares Resektoskop nach einem der Ansprüche 1 bis 10,
**dadurch gekennzeichnet,**
**dass** der Isoliereinsatz (8, 8') aus einer Keramik ausgebildet ist und
**dass** die Neutralelektrode (5, 5') aus einer metallisierten Keramik ausgebildet ist.

12. Bipolares Resektoskop nach einem der Ansprüche 1 bis 11,
**dadurch gekennzeichnet,**
**dass** der Schaft (2, 2') aus einem elektrisch leitenden Metall ausgebildet ist.

13. Bipolares Resektoskop nach einem der Ansprüche 1 bis 12,
**dadurch gekennzeichnet,**
**dass** der Schaft (2, 2') mindestens auf seiner Außenfläche mit einem elektrisch nicht leitenden Material beschichtet ist.

14. Bipolares Resektoskop nach einem der Ansprüche 1 bis 13,
**dadurch gekennzeichnet,**
**dass** der Schaft (2, 2') in einem Außenschaft (40, 40') einsteckbar ist und
**dass** zwischen dem Außenschaft (40, 40') und dem eingesetzten Schaft ein Absaugkanal (44, 44') gebildet wird.

## Claims

1. A bipolar resectoscope (1,1) comprising
- a shaft (2, 2') having an insulating insert (8, 8') made of an electrically non-conductive material at its distal end (7), where the insulating insert (8, 8') has at its exposed, distal end (7) facing away from the shaft (2, 2') an inlet area (36, 36'), arranged in vertical direction below its longitudinal axis (33), for a longitudinally displaceable cutting electrode (4), and the insulating insert (8, 8') has an exposed electrically conductive area of a neutral electrode (5, 5') which is electrically connected to a passive electrode terminal at the proximal end (7) of the shaft (2, 2'),
- a working element (3) mountable in the shaft (2, 2') in which the active cutting electrode (4) can be arranged in a longitudinally displaceable manner, said active cutting electrode (4) being connected at its proximal end (19) facing away from the distal end (23) to an active electrode terminal at the proximal end (11) of the working element (3), and said working element (3) having an optical guide tube (10) to accommodate an optical unit (6) which can be inserted in the working element (3), and
- the active cutting electrode (4)
**characterized in that**
the area of the passive neutral electrode (5, 5') exposed toward the outside and transverse to the longitudinal axis (33) of the insulating insert (8, 8') forms a continuously electrically conductive roof section (32, 32') of the insulating insert (8, 8') toward the inside,
that the exposed portion of the passive neutral electrode (5, 5') is limited to the roof section (32, 32') of the insulating insert (8, 8') in the inlet area (36, 36'),
that the roof section is arranged transverse to the longitudinal axis (33) of the insulating insert in vertical direction above the inlet area (36),
that the roof section (32, 32') extends in the direction of the longitudinal axis (33) over the inlet area (36) of the insulating insert (8),
that the insulating insert (8, 8') in the inlet area (36, 36') has on its inside wall (38, 38') two exposed electrically conductive contact surfaces (39, 39') of the neutral electrode (5, 5') facing each other and running parallel to the longitudinal axis (33), which, in a configuration with a cutting electrode (4) mounted inside the shaft (2, 2'), are in metallic electrically conductive contact with the neutral electrode (5, 5') by means of one adjacent contact tube (24) each of a fork (26) having two parallel electrically conductive contact tubes (24) and opening a cutting loop (25) of the cutting electrode (4), and
that the electrically conductive contact tubes (24) are electrically isolated from the fork (26).

2. The bipolar resectoscope according to claim 1,
**characterized in that**
the exposed electrically conductive contact surfaces (39, 39') of the neutral electrode (5, 5') of the insulating insert (8, 8') outside of the roof section (32, 32') toward the proximal end (31, 31') of the insulating insert (8, 8') are formed by circumferential contact surfaces (39, 39') of the neutral electrode (5, 5') that are exposed toward the inside.

3. The bipolar resectoscope according to claim 1 or 2,
**characterized in that**
the electrically conductive contact tubes (24) have longitudinal openings (28) facing an inserted optical unit (6) and exposing the insulating layer (29) below.

4. The bipolar resectoscope according to claim 2 or 3,
**characterized in that**
that in the area of the fork (26) the cutting electrode (4) has a guide tube (30) that is routed at the distal end (23) of the optical unit (6) inserted in the working element (3) and is electrically isolated from the contact tubes (24) and the fork (26).

5. The bipolar resectoscope according to claim 4,
**characterized in that**
the guide tube (30) of the cutting electrode (4) is made of an electrically non-conductive material.

6. The bipolar resectoscope according to claims 1 to 5,
**characterized in that**
in its circumference outside of the roof section (32, 32') and the contact surfaces (39, 39') or contact surface (39, 39'), the neutral electrode (5, 5') has an electrically non-conductive coating both on its outer and inner surface.

7. The bipolar resectoscope according to claims 1 to 6,
**characterized in that**
on its front face located at the distal end (35, 35') of the insulating insert (8, 8'), outside of the roof section (32, 32'), the neutral electrode (5, 5') has an electrically non-conductive coating.

8. The bipolar resectoscope according to claims 1 to 7,
**characterized in that**
in longitudinal direction to the distal end (35, 35') of the shaft (2, 2'), the neutral electrode (5, 5') of the insulating insert (8, 8') is designed as non-conductive.

9. The bipolar resectoscope according to claims 1 to 8,
**characterized in that**
at least the inlet area (36, 36') of the insulating insert (8, 8') is protected by zirconium dioxide ceramic.

10. The bipolar resectoscope according to claims 1 to 9,
**characterized in that**
the insulating insert (8, 8') is made of a plastic material and the neutral electrode (5, 5') is made of metal.

11. The bipolar resectoscope according to claims 1 to 10,
**characterized in that**
the insulating insert (8) is made of a ceramic material and the neutral electrode (5) is made of metalized ceramic.

12. The bipolar resectoscope according to claims 1 to 11,
**characterized in that**
the shaft (2, 2') is made of an electrically conductive metal.

13. The bipolar resectoscope according to claims 1 to 12,
**characterized in that**
the shaft (2, 2') is coated with an electrically non-conductive material at least on its outer surface.

14. The bipolar resectoscope according to claims 1 to 13,
**characterized in that**
the shaft (2, 2') can be inserted into an outer shaft (40, 40') and a suction channel (44, 44') is formed between the outer shaft (40, 40') and the inserted shaft.

## Revendications

1. Résectoscope bipolaire (1, 1'), comprenant
- une tige (2, 2') présentant à son extrémité distale (7) un insert isolant (8, 8') constitué d'un matériau non électroconducteur, sachant que ledit insert isolant (8, 8') comporte à son extrémité distale (7) exposée, opposée à la tige (2, 2'), une zone d'entrée (36, 36'), disposée en direction verticale en dessous de son axe longitudinal (33), pour une électrode de résection (4), pouvant être disposée dans la tige (2, 2') de manière à coulisser longitudinalement, et que ledit insert isolant (8, 8') présente une zone exposée électroconductrice d'une électrode neutre (5, 5') qui entre en liaison électrique avec une borne d'électrode passive à l'extrémité proximale (7) de la tige (2,2'),
- un élément de travail (3) pouvant être disposé dans la tige (2, 2'), dans lequel l'électrode de résection (4) active peut être placée de manière à coulisser longitudinalement, entrant en liaison électrique à son extrémité proximale (19) opposée à son extrémité distale (23) avec une borne d'électrode active à l'extrémité proximale (11) de l'élément de travail (3), et qui présente un tube de guidage optique (10) pour recevoir un élément optique (6) pouvant être inséré dans l'élément de travail (3), et
- l'électrode de résection (4) active,
**caractérisé en ce**
**que** la zone de l'électrode neutre (5, 5') passive, exposée vers l'extérieur dans le sens transversal par rapport à l'axe longitudinal (33) de l'insert isolant (8, 8'), forme une zone de toit (32, 32') de l'insert isolant (8, 8') électroconductrice en continu vers l'intérieur, que la zone exposée de l'électrode neutre (5, 5') passive est limitée dans la zone d'entrée (36, 36') à la zone de toit (32, 32') de l'insert isolant (8, 8'), que la zone de toit est disposée dans la direction verticale au-dessus de la zone d'entrée (36, 36') dans le sens transversal par rapport à l'axe longitudinal (33) de l'insert isolant, et
**que** la zone de toit (32, 32') s'étend dans la direction de l'axe longitudinal (33) au-dessus de la zone d'entrée (36, 36') de l'insert isolant (8, 8'),
**que** l'insert isolant (8, 8') présente dans la zone d'entrée (36, 36'), sur sa paroi interne (38, 38'), deux surfaces de contact (39, 39') exposées électroconductrices de l'électrode neutre (5, 5'), situées l'une en face de l'autre, qui s'étendent parallèlement à l'axe longitudinal (33) et qui, dans le cas d'une l'électrode de résection (4) disposée dans la tige (2, 2'), sont en contact métallique électroconducteur avec l'électrode neutre (5, 5') moyennant, respectivement, un petit tube de contact (24) adjacent d'une fourche (26) présentant deux petits tubes de contact (24) parallèles électroconducteurs et ouvrant une boucle coupante (25) de l'électrode de résection (4), et que les petits tubes de contact (24) électroconducteurs sont isolés électriquement par rapport à la fourche (26).

2. Résectoscope bipolaire selon la revendication 1,
**caractérisé en ce**
**que** les surfaces de contact (39, 39') exposées électroconductrices de l'électrode neutre (5, 5') de l'insert isolant (8, 8') sont formées par une surface de contact (39, 39') périphérique, exposée vers l'intérieur, en dehors de la zone de toit (32, 32'), vers l'extrémité proximale (31, 31 ') de l'insert isolant (8, 8').

3. Résectoscope bipolaire selon la revendication 1 ou 2,
**caractérisé en ce**
**que** les petits tubes de contact (24) électroconducteurs présentent des ouvertures longitudinales (28) dirigées vers un élément optique (6) introduit, qui dégagent la couche isolante (29) sous-jacente.

4. Résectoscope bipolaire selon la revendication 2 ou 3,
**caractérisé en ce**
**que** l'électrode de résection (4) comporte, dans la zone de la fourche (26), un tube de guidage (30) guidé à l'extrémité distale (23) de l'élément optique (6) introduit dans l'élément de travail (3), ledit tube de guidage étant isolé électriquement par rapport aux petits tubes de contact (24) et à la fourche (26).

5. Résectoscope bipolaire selon la revendication 4,
**caractérisé en ce**
**que** le tube de guidage (30) de l'électrode de résection (4) est constitué d'un matériau non électroconducteur.

6. Résectoscope bipolaire selon l'une des revendications 1 à 5,
**caractérisé en ce**
**que** l'électrode neutre (5, 5') est munie d'un revêtement non électroconducteur, à la fois sur sa surface extérieure et sur sa surface intérieure, sur sa périphérie en dehors de la zone de toit (32, 32') et des surfaces de contact (39, 39') ou de la surface de contact (39, 39').

7. Résectoscope bipolaire selon l'une des revendications 1 à 6,
**caractérisé en ce**
**que** l'électrode neutre (5, 5') est munie d'un revêtement non électroconducteur à sa surface frontale disposée à l'extrémité distale (35, 35') de l'insert isolant (8, 8') en dehors de la zone de toit (32, 32').

8. Résectoscope bipolaire selon l'une des revendications 1 à 7,
**caractérisé en ce**
**que** l'électrode neutre (5, 5') de l'insert isolant (8, 8') est réalisée de façon non conductrice dans le sens longitudinal par rapport à l'extrémité distale (35, 35') de la tige (2, 2').

9. Résectoscope bipolaire selon l'une des revendications 1 à 8,
**caractérisé en ce**
**qu'**au moins la zone d'entrée (36, 36') de l'insert isolant (8, 8') est protégée par un matériau céramique à base de dioxyde de zirconium.

10. Résectoscope bipolaire selon l'une des revendications 1 à 9,
**caractérisé en ce**
**que** l'insert isolant (8, 8') est constitué d'un matériau synthétique et
**que** l'électrode neutre (5, 5') est constituée d'un matériau métallique.

11. Résectoscope bipolaire selon l'une des revendications 1 à 10,
**caractérisé en ce**
**que** l'insert isolant (8, 8') est en céramique et
**que** l'électrode neutre (5, 5') est en céramique métallisée.

12. Résectoscope bipolaire selon l'une des revendications 1 à 11,
**caractérisé en ce**
**que** la tige (2, 2') est constituée d'un matériau électroconducteur.

13. Résectoscope bipolaire selon l'une des revendications 1 à 12,
**caractérisé en ce**
**que** la tige (2, 2') est recouverte d'un matériau non électroconducteur au moins sur sa surface extérieure.

14. Résectoscope bipolaire selon l'une des revendications 1 à 13,
**caractérisé en ce**
**que** la tige (2, 2') peut être enfichée dans une tige externe (40, 40') et qu'un canal d'aspiration (44, 44') est formé entre la tige externe (40,40') et la tige insérée.
